# EUROPEAN PATENT APPLICATION

(11) **EP 1 284 148 A2**
(43) Date of publication of application: **19.02.2003**
(21) Application number: 02015976.0
(22) Date of filing: 18.07.2002
(51) Int. Cl.: A61N 1/05

(54) **Procedure for platinum coating of titanium cardiac electrodes**

(30) Priority: 06.08.2001 IT PD20010199
(71) Applicant: Medico S.p.a., 35121 Padova (IT)
(72) Inventor: Snichelotto, Eugenio, 35121 Padova (IT); Baraldo, Marco, 35121 Padova (IT)
(74) Representative: Gustorf, Gerhard, Dipl.-Ing.

(57) **Abstract**

The invention is a new procedure for platinum coating of titanium cardiac electrodes, comprising the spark erosion of the surface of the titanium electrode and the subsequent electrochemical deposition of platinum on the electrode. The spark erosion of the electrode surface is performed in a solution of methanol and sodium chloride and, after the electrodeposition of platinum, the electrode is subjected to the action of a regulated flame in order to permanently fix the platinum to the titanium electrode.

## Description

### FIELD OF THE INVENTION

This patent concerns medical electrodes for intracardiac use and in particular it concerns a new procedure for platinum coating of titanium cardiac electrodes.

### PRIOR ART

At present medical electrodes for intracardiac use are made of titanium and/or titanium with titanium nitride coating.

The electrodes produced in this way have the following problems and disadvantages.

The electrodes currently used remain partly polarised after stimulation, with the result that the stimulation signal is prolonged and distorted.

The electrodes currently used have a relatively high pacing threshold, which requires gauging of the pacing circuit in order to compensate for said threshold.

### DESCRIPTION

To solve all the above problems, a new procedure for platinum coating of titanium cardiac electrodes has been studied and implemented.

The aim of the new procedure is to securely and uniformly fix the platinum coating on the titanium of the electrode.

Another purpose of the new procedure is to join platinum to the titanium of the electrode in a uniform manner, in order to eliminate any capacitive and resistance effect.

Procedure for platinum coating of titanium cardiac electrodes comprising initial spark erosion of the electrode surface, subsequent electrochemical deposition of platinum on the titanium surface which has been given a rough micro-finish, and final hot-fixing by means of regulated flame.

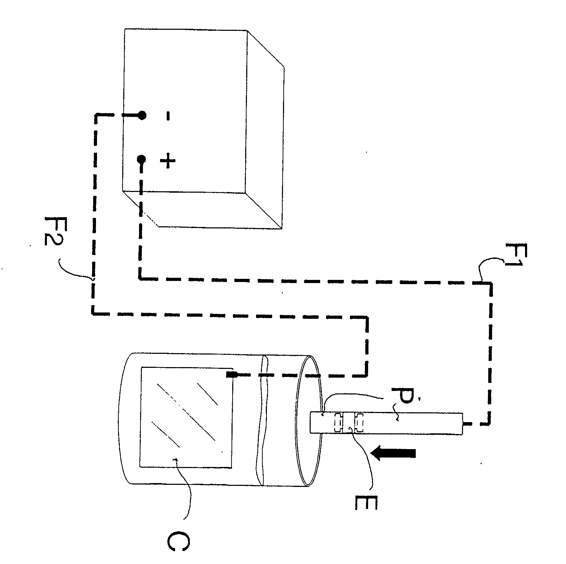

The characteristics of the new procedure for platinum coating of titanium cardiac electrodes will be clarified by the following description, with reference to the drawing attached as a non-restrictive example.

The titanium electrode (E) is produced in the necessary form and dimensions by means of the known procedures which are not discussed here.

The surface of the titanium electrode (E) is too smooth for the optimal deposition of platinum.

The electrode (E) is connected by an electric wire (F1) and the surfaces (P) not to be coated with platinum are protected.

The electrode (E) prepared as above is immersed in a solution of methanol and sodium chloride in which a titanium counter electrode (C) has been immersed, connected by a second electric wire (F2).

By applying electric current to the two wires (F1, F2), spark erosion is performed on the exposed surface of the electrode (E), giving it a rough micro-finish. Said finish provides a better and safer surface for the adhesion of platinum.

Once spark erosion has been performed, the titanium electrode (E) is electrochemically coated with a layer of platinum. This second electrochemical phase deposits on the rough micro-finish surface of the electrode (E) a layer of platinum with thickness depending on the duration of the electrochemical deposition process on the electrode (E).

Lastly, the electrode (E) coated with the layer of platinum is exposed to a regulated flame for permanent fixing of the platinum on the electrode.

The procedure for platinum coating of titanium cardiac electrodes described above produces cardiac electrodes with improved characteristics.

The rough micro-finish obtained on the surface of the electrode permits the improved adhesion of platinum on the electrode (E).

The hot-fixing of platinum on the electrode (E) permanently joins platinum to the surface of the electrode (E) and ensures uniformity in both the outer platinum surface and in the adhesion of platinum to the surface of the titanium electrode (E).

The electrode thus obtained consequently has a very low pacing threshold and is free from capacitive effects and post-stimulation polarisation.

With reference to the above description and the attached drawing, the following claims are therefore expressed.

## Claims

1. Procedure for platinum coating of titanium cardiac electrodes, **characterised in that** it comprises the spark erosion of the surface of the titanium electrode (E) and the subsequent electrochemical deposition of platinum on the electrode (E).

2. Procedure for platinum coating of titanium cardiac electrodes according to claim 1, **characterised in that** the spark erosion of the electrode surface (E) is performed in a solution of methanol and sodium chloride.

3. Procedure for platinum coating of titanium cardiac electrodes according to claims 1 and 2, **characterised in that** the electrode (E), after the electrodeposition of platinum, is subjected to the action of a regulated flame in order to permanently fix the platinum to the titanium electrode.
